(19)

**Europäisches Patentamt**

**European Patent Office**

**Office européen des brevets**

(11) **EP 4 506 950 A1**

(12) **EUROPEAN PATENT APPLICATION**
published in accordance with Art. 153(4) EPC

(43) Date of publication:
**12.02.2025 Bulletin 2025/07**

(21) Application number: **23807800.0**

(22) Date of filing: **21.04.2023**

(51) International Patent Classification (IPC):
*G16C 20/70* (2019.01)   *G16C 20/50* (2019.01)
*G16C 20/10* (2019.01)   *G16C 20/30* (2019.01)
*G16C 20/90* (2019.01)   *G06N 3/04* (2023.01)
*G16C 20/62* (2019.01)   *G16C 60/00* (2019.01)

(52) Cooperative Patent Classification (CPC):
**G06N 3/04; G16C 20/10; G16C 20/30; G16C 20/50; G16C 20/62; G16C 20/70; G16C 20/90; G16C 60/00**

(86) International application number:
**PCT/KR2023/005431**

(87) International publication number:
**WO 2023/224277 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.05.2022   KR 20220061619**
**10.01.2023   KR 20230003635**

(71) Applicants:
• **LG Management Development Institute Co., Ltd.**
**Seoul 07336 (KR)**
• **Korea Advanced Institute of Science and Technology**
**Daejeon 34141 (KR)**

(72) Inventors:
• **KIM, Kiyoung**
**Seoul 07584 (KR)**
• **LEE, Honglak**
**Seoul 07796 (KR)**
• **HAN, Sehui**
**Seoul 07665 (KR)**
• **LEE, Moontae**
**Seoul 07796 (KR)**
• **JEONG, Daewoong**
**Seoul 07523 (KR)**
• **JUNG, Yousung**
**Daejeon 34141 (KR)**
• **NOH, Juhwan**
**Daejeon 34141 (KR)**

(74) Representative: **BCKIP Part mbB**
**MK1**
**Landsbergerstraße 98, 3.Stock**
**80339 München (DE)**

(54) **AUTOENCODING DEVICE FOR SYNTHESIZABLE MOLECULE GENERATION MODEL TAKING INTO CONSIDERATION MOLECULAR STRUCTURE CONDITIONS AND MOLECULE GENERATION METHOD USING SAME**

(57)    An autoencoding device for a synthesizable molecule generative model according to an exemplary embodiment of the present disclosure may include a memory storing chemical reaction data, chemical reaction learning information, and molecular structure information; and a processor configured to learn a neural network that implements a molecular generative model based on the chemical reaction data, the chemical reaction learning information, and the structural information, wherein the molecular generative model may include: a reaction sequence encoder that generates a latent space based on the chemical reaction learning information; a seed molecule encoder that generates an embedding space based on seed structural information for the seed molecule that is a final product of the chemical reaction; and a decoder that outputs a reconstruction reaction sequence by decoding a reaction molecule predictive neural network and a reaction template prediction model generated from the latent space and the embedding space.

EP 4 506 950 A1

[FIG. 1]

## Description

### Technical Field

The present disclosure relates to an autoencoding device for a molecular generative model and a molecule generation method. More specifically, the present disclosure relates to an autoencoding device for a synthesizable molecule generative model considering molecular structural conditions and method of generating a molecule using the same.

### Background

Recently, various concepts and learning models are being developed in the field of artificial intelligence technology, and research on data prediction using these is actively being conducted. When predicting data based on artificial intelligence-based neural networks, learning or prediction algorithms for learning models are being developed to derive results with high prediction probability.

Molecular creation models are algorithms that have recently been gaining attention in the field of new material development because they may reverse engineer molecules with desired properties. However, existing molecular creation models have limitations in that they do not consider the synthesizable nature of molecules. Therefore, a new methodology is required that may simultaneously generate chemical structures and synthetic routes with desired properties.

### Detailed Description of the Invention

### Technical Problem

The purpose of the embodiments disclosed in the present disclosure is to provide an autoencoding device for a synthesizable molecule generative model considering molecular structural conditions and a molecule generation method using the same.

The problems to be solved by the present disclosure are not limited to the problems mentioned above, and other problems not mentioned will be clearly understood by those skilled in the art from the description below.

### Means to Solve the Problem

According to an exemplary embodiment of the present disclosure for achieving the above-described technical problem, an autoencoding device for a synthesizable molecule generative model may include a memory storing chemical reaction data, chemical reaction learning information, and molecular structure information; and a processor configured to learn a neural network that implements a molecular generative model based on the chemical reaction data, the chemical reaction learning information, and the structural information, wherein the molecular generative model may include: a reaction sequence encoder that generates a latent space based on the chemical reaction learning information; a seed molecule encoder that generates an embedding space based on seed structural information for the seed molecule that is a final product of the chemical reaction; and a decoder that outputs a reconstruction reaction sequence by decoding a reaction molecule predictive neural network and a reaction template prediction model generated from the latent space and the embedding space.

In addition, a method of generating a molecular model using a computing device according to an exemplary embodiment of the present disclosure for achieving the above-described technical problem may include obtaining a latent space comprising chemical reaction learning information; obtaining an embedding space comprising structural information but omitting synthetic path information of a molecule; sampling similar molecules from the latent space and the embedding space through a generative model, and generating candidate molecules; generating a synthetic pathway between molecules with seed structural information; and outputting a seed molecular model that comprises the seed structural information and the synthetic pathway.

### Effects of the Invention

According to the aforementioned problem solving means of the present disclosure, by forming and implementing a chemical bonding space composed of synthesizable compounds, a chemical reaction database capable of generating compounds having high synthesizable properties and at the same time required properties may be constructed.

In addition, according to the aforementioned problem solving means of the present disclosure, the time for filtering out synthesizable molecules among the new substances generated by the molecular generative model and deriving the synthetic path may be shortened through a variational autoencoder-based machine learning methodology capable of simultaneously generating the chemical structure of a molecule having required properties and a chemical reaction path (synthetic path).

The effects of the present disclosure are not limited to the effects mentioned above, and other effects not mentioned will be clearly understood by those skilled in the art from the description below.

**Brief Description of the Drawings**

FIG. 1 is a schematic diagram schematically illustrating a molecular generative model according to an exemplary embodiment of the present disclosure.

FIG. 2 is a flow chart illustrating an operation method of an autoencoding device for a synthetic molecule generative model according to an exemplary embodiment of the present disclosure.

FIG. 3 is a schematic diagram illustrating a process for generating chemical reaction data for a synthetic molecule generative model according to an exemplary embodiment of the present disclosure.

FIG. 4 is a flow chart illustrating a method for generating chemical reaction data for a synthetic molecule generative model according to an exemplary embodiment of the present disclosure.

FIG. 5 is a schematic diagram illustrating an autoencoding device for a syntheticable molecule generative model according to an exemplary embodiment of the present disclosure.

FIG. 6 is a conceptual diagram detailing the operation of an autoencoding device for a synthetic molecule generative model according to an exemplary embodiment of the present disclosure.

FIG. 7 is a conceptual diagram illustrating in detail the operation of an autoencoding device for a synthetic molecule generative model according to an exemplary embodiment of the present disclosure.

FIG. 8 is a conceptual diagram illustrating in detail the operation of an autoencoding device for a synthesizable molecule generative model according to an exemplary embodiment of the present disclosure.

FIG. 9 is a conceptual diagram illustrating the operation of an autoencoding device for a synthesizable molecule generative model according to an exemplary embodiment of the present disclosure.

FIG. 10 is a graph showing the performance of an autoencoding device for a synthesizable molecule generative model according to an exemplary embodiment of the present disclosure.

FIG. 11 is a graph showing the performance of an autoencoding device for a synthesizable molecule generative model according to an exemplary embodiment of the present disclosure.

FIG. 12 is a conceptual diagram illustrating the performance of an autoencoding device for a synthesizable molecule generative model according to an exemplary embodiment of the present disclosure.

**Embodiments for the Implementation of the Invention**

Throughout the present disclosure, identical reference numerals refer to the same components. The present disclosure does not describe all elements of the embodiments, and general contents within the technical field to which the present disclosure or overlapping details between embodiments pertains are omitted. The terms "unit," "module," "member," and "block" used in the specification may be implemented in either software or hardware, and according to embodiments, it is also possible for a plurality of "units," "modules," "members," and "blocks" to be implemented as a single component, or for one "unit," "module," "member," and "block" to include a plurality of components.

Throughout this specification, when a part is described as being "connected" to another part, this includes not only cases where they are directly connected, but also cases where they are indirectly connected, and an indirect connection includes a connection via a wireless communications network.

In addition, when a part is said to "include" a certain component, this means that it may further include other components rather than excluding other components, unless specifically stated to the contrary.

Throughout this specification, when a member is said to be located "on" another member, this includes not only the case where the member is in contact with the other member, but also the case where another member exists between the two members.

The terms such as first, second are used to distinguish one component from another component, and the components are not limited by the aforementioned terms.

Singular expressions include plural expressions unless the context clearly indicates otherwise.

The identification symbols in the each step are used for the convenience of explanation and do not explain the order of the each step, and each step may be performed in a different order than specified unless the context clearly indicates a specific order.

Hereinafter, the principles of operation and embodiments of the present disclosure will be described with reference to the accompanying drawings.

Before the explanation, the meaning of terms used in this disclosure will be briefly explained. However, since the explanation of terms is intended to help the understanding of this specification, it should be noted that they are not used in a meaning that limits the technical idea of this disclosure unless explicitly stated to limit the disclosure.

In this specification, the terms neural network, artificial neural network, and network function are often used interchangeably.

Also, throughout this specification, the terms neural network, neural network, and network function may be used interchangeably. A neural network may be composed of a set of interconnected computational units, which may generally be referred to as "nodes." These "nodes" may also be referred to as "neurons." A neural network is composed of at least two or more nodes. The nodes (or neurons) constituting the neural networks may be interconnected by one or more "links."

The present disclosure discloses a computing device as an example of an electronic processing device. For example, the computer device may be a server, a workstation, or the like, as an electronic processing device that processes information by performing communication with an external device, such as an application server, a computing server, a database server, a file server, a game server, a mail server, a proxy server, and a web server. Alternatively, the computing device may include a notebook, desktop, laptop, tablet PC, slate PC, etc. equipped with a web browser.

A computing device may include a memory, a processor, an input unit, and an output unit. The artificial intelligence-related function according to the present disclosure is operated through the processor and the memory.

The memory may store at least one instruction and various data required for artificial intelligence learning. According to one embodiment of the present disclosure, the memory may include various types of storage media including at least one of a flash memory type, a hard disk type, a multimedia card micro type, a card type memory (for example, an SD or XD memory, etc.), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disk, an optical disk, resistive memory cells such as ReRAM (resistive RAM), a PRAM (phase change RAM), an MRAM (magnetic RAM), an MRAM (Spin-Transfer Torgue MRAM), a CBRAM (conductive bridging RAM), and a FeRAM (ferroelectric RAM).

The processor may be configured to perform at least one instruction for artificial intelligence learning. The processor of the present disclosure may be configured with one or more cores, and may include a processor for data analysis and deep learning, such as a neural processing unit (NPU), a central processing unit (CPU), a general purpose graphics processing unit (GPGPU), and a tensor processing unit (TPU) of a computing device. The processor may read a computer program stored in a memory and perform data processing for machine learning according to an embodiment of the present disclosure.

The processor may be a general-purpose processor such as a CPU, an AP, a DSP (Digital Signal Processor), a graphics-only processor such as a GPU, a VPU (Vision Processing Unit), or an AI-only processor such as an NPU. One or more processors are controlled to process input data according to predefined operation rules or AI models stored in the memory 130. Alternatively, when one or more processors are AI-only processors, the AI-only processor may be designed with a hardware structure specialized for processing a specific AI model.

According to one embodiment of the present disclosure, a processor may perform operations for learning a neural network. The processor may perform calculations for learning a neural network, such as processing input data for learning in deep learning (DL), extracting features from input data, calculating errors, and updating weights of a neural network using backpropagation. At least one of the NPU, CPU, GPGPU, and TPU of the processor may process learning of a network function. For example, the CPU and GPGPU may together process learning of a network function and classification of data using a network function. In addition, in one embodiment of the present disclosure, processors of a plurality of computing devices may be used together to process learning of a network function and classification of data using a network function. In addition, a computer program executed in a computing device according to one embodiment of the present disclosure may be a CPU, GPGPU, or TPU executable program.

The predefined operation rules or artificial intelligence models are characterized by being created through learning. Here, being created through learning means that the basic artificial intelligence model is learned by using a plurality of learning data by a learning algorithm, thereby creating a predefined operation rules or artificial intelligence model set to perform a desired characteristic (or purpose). Such learning may be performed in the device itself on which the artificial intelligence according to the present disclosure is performed, or may be performed through a separate server and/or system. Examples of the learning algorithm include supervised learning, unsupervised learning, semi-supervised learning, or reinforcement learning, but are not limited to the examples described above.

The AI model may be a single AI model or may be implemented as multiple AI models. The AI model may be composed of a neural network (or an artificial neural network) and may include a statistical learning algorithm that mimics biological neurons in machine learning and cognitive science. The neural network may refer to a model in which artificial neurons (nodes) that form a network by combining synapses change the binding strength of synapses through learning, thereby having a problem-solving ability. The neurons of the neural network may include a combination of weights or biases. The neural network may include one or more layers composed of one or more neurons or nodes. For example, the device 100 may include an input layer, a hidden layer, and an output layer. The neural network constituting the device 100 may infer a desired result (output) from an arbitrary input (input) by changing the weights of neurons through learning.

The processor may create a neural network, train (or learn) a neural network, perform computations based on received input data, generate information signals based on the results of the computations, or retrain a neural network. Neural network models include CNN (Convolution Neural Network), R-CNN (Region with Convolution Neural Network), RPN (Region Proposal Network), RNN (Recurrent Neural Network), S-DNN (Stacking-based deep Neural Network), S-SDNN (State-Space Dynamic Neural Network), Deconvolution Network, DBN (Deep Belief Network), and RBM (Restrcted Boltzman Machine) such as GoogleNet, AlexNet, VGG Network, etc. ), Fully Convolutional Network, LSTM (Long Short-Term Memory) Network, Classification Network, Generative Modeling, eXplainable AI, Continual AI, Representation Learning, AI for Material Design, BERT for natural language processing, SP-BERT, MRC/QA, Text Analysis, Dialog System, GPT-3, GPT-4, Visual Analytics for vision processing, Visual Understanding, Video Synthesis, Anomaly Detection for ResNet data intelligence, Time-Series Forecasting, Optimization, It may include various types of models such as Recommendation, Data Creation, etc., but is not limited thereto. The processor may include one or more processors for performing operations according to models of the neural network. For example, the neural network may include a deep neural network.

Neural networks include CNN (Convolutional Neural Network), RNN (Recurrent Neural Network), perceptron, multilayer perceptron, FF (Feed Forward), RBF (Radial Basis Network), DFF (Deep Feed Forward), and LSTM. (Long Short Term Memory), GRU (Gated Recurrent Unit), AE (Auto Encoder), VAE (Variational Auto Encoder), DAE (Denoising Auto Encoder), SAE (Sparse Auto Encoder), MC (Markov Chain), HN (Hopfield) Network), BM (Boltzmann Machine), RBM (Restricted Boltzmann Machine), DBN (Depp Belief Network), DCN (Deep Convolutional Network), DN (Deconvolutional Network), DCIGN (Deep Convolutional Inverse Graphics Network), GAN (Generative Adversarial Network)), Liquid State Machine (LSM), Extreme Learning Machine (ELM), It may include ESN (Echo State Network), DRN (Deep Residual Network), DNC (Differentiable Neural Computer), NTM (Neural Turning Machine), CN (Capsule Network), KN (Kohonen Network) and AN (Attention Network). It will be appreciated by those skilled in the art that this may include any neural network, but is not limited thereto.

The network unit according to one embodiment of the present disclosure may enable communication between a plurality of computing devices so that operations for determining a user control operation range or learning a model may be performed in a distributed manner on each of the plurality of computing devices. The network unit may enable communication between a plurality of computing devices so that operations for autism diagnosis or model learning using a network function may be processed in a distributed manner. The network unit according to one embodiment of the present disclosure may operate based on any form of wired and wireless communication technology currently in use and implemented, such as short-distance (short-distance), long-distance, wired, and wireless, and may also be used in other networks.

An output unit according to one embodiment of the present disclosure may display a user interface (UI) for providing a result of determining a user control operation range and a judgment. The output unit may output any form of information generated or determined by a processor and any form of information received by a network unit. In one embodiment of the present disclosure, the output unit may include at least one of a liquid crystal display (LCD), a thin film transistor-liquid crystal display (TFT LCD), an organic light-emitting diode (OLED), a flexible display, and a three-dimensional display (3D display). Some of these display modules may be configured as transparent or light-transmitting so that the outside may be viewed therethrough. This may be referred to as a transparent display module, and representative examples of the transparent display module include, but are not limited to, TOLED (Transparent OLED).

An input unit according to one embodiment of the present disclosure may receive a user input. The input unit may have keys and/or buttons on a user interface for receiving a user input, or physical keys and/or buttons. A computer program for controlling a display according to embodiments of the present disclosure may be executed according to a user input through the input unit.

The input unit according to embodiments of the present disclosure may receive a signal by detecting a user's button operation or touch input, or may receive a user's voice or motion through a camera or microphone and convert it into an input signal. For this purpose, speech recognition technology or motion recognition technology may be used.

The input unit according to embodiments of the present disclosure may be implemented as an external input device connected to an external system. For example, the input device may be at least one of a touchpad, a touch pen, a keyboard, or a mouse for receiving user input, but this is only an example and is not limited thereto.

An input unit according to one embodiment of the present disclosure may recognize a user touch input. The input unit according to one embodiment of the present disclosure may have the same configuration as the output unit. The input unit may be configured with a touch screen implemented to receive a user's selection input. The touch screen may use any one of a contact-type electrostatic capacitance method, an infrared optical sensing method, a surface-acoustic (SAW) method, a piezoelectric method, and a resistive film method. The detailed description of the touch screen described above is only an example according to one embodiment of the present disclosure, and various touch screen panels may be employed in a computing device. The input unit configured with a touch screen may include a touch sensor. The touch sensor may be configured to convert a change in pressure applied to a specific portion of the input unit or electrostatic capacitance occurring in a specific portion of the input unit into an electrical input signal. The touch sensor may be configured to detect not only the position and area of the touch, but also the pressure at the time of the touch. When there is a touch input to the touch sensor, a corresponding signal(s) is sent to the touch controller. The touch controller may process the signal(s) and then transmit corresponding data to the processor, thereby enabling the processor to recognize which area of the input section has been touched, etc.

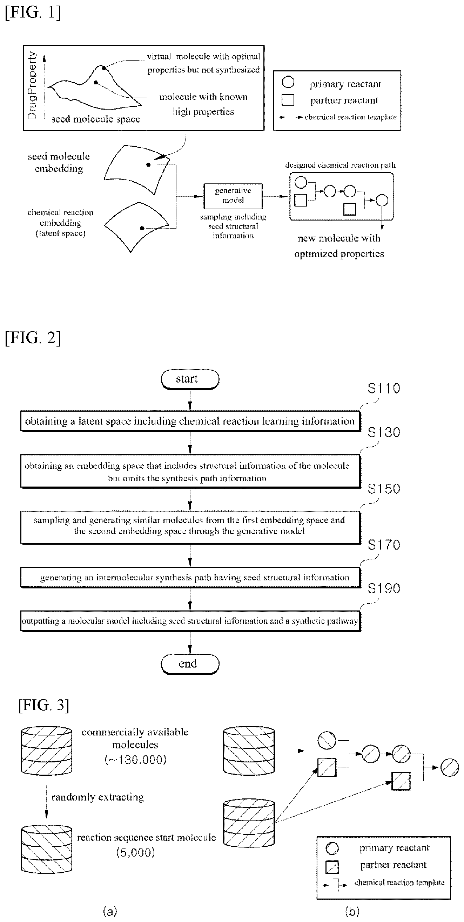

FIG. 1 is a schematic diagram schematically illustrating a molecular generative model according to an exemplary embodiment of the present disclosure. The molecular generative model of the present disclosure may be implemented through the computing device described above.

AI and machine learning-based new material design methods are technologies that may accelerate material design in various fields such as drugs, OLEDs, and solar cells, and performance verification procedures are being carried out through the discovery and synthesis of new materials by applying machine learning methodologies in various fields. In particular, in the case of generative models, it is a methodology that may simulate the distribution of data, and has the characteristic of being able to generate new data from the learned probability distribution. In the case of organic molecules, various types of molecular generative models have been proposed using string-based expressor or molecular graph expressor, such as SMILES (Simplified Molecular Input Line Entry System), and additionally, models that may generate molecules with desired properties by utilizing information on physical properties have been reported.

In the case of existing molecular generative models, there is a disadvantage in that there is no explicit information about the synthetic possibility directly related to experimental verification. Therefore, to solve this problem, a method needs to be proposed to extract the features of existing known molecules and obtain the degree of synthetic possibility or to reversely estimate the synthetic path of a given molecule.

Illustrative embodiments of the present disclosure present a methodology for designing directly synthesizable molecules via generative models, and specifically propose novel models in which information about chemical reaction pathways is learned in addition to information about individual molecular structures.

Referring to FIG. 1, the concept of a molecular generative model according to the present disclosure is explained. The molecular generative model according to an exemplary embodiment of the present disclosure utilizes structural information of a molecule desired by a user (i.e., seed molecule embedding) as a condition along with a latent space (i.e., reaction embedding) in which information about a chemical reaction is learned, thereby maintaining (structure-preserving) chemical structural information of a specific molecule and generating a reaction path (designed reaction path) or a synthesis pathway of the molecule as a result of sampling.

In the case of generative models related to existing chemical reactions, since new chemical reactions are randomly obtained from the learned latent space, structural information specifically desired by the user is not reflected, and additional post-processing work is required for this, which has a disadvantage. In contrast, in the case of the molecular generative model according to the present disclosure, the structural information of molecules (synthetically unknown optimized molecules) that have optimized properties but whose synthetic paths are not known through the existing generative model are used as conditions to generate chemical reactions, so that the user may simultaneously generate synthetic paths while utilizing structural information specifically desired by the user.

According to the present disclosure, by forming and implementing a chemical bonding space composed of synthesizable compounds, a chemical reaction database capable of generating compounds having high synthesizable properties and at the same time required properties may be constructed.

In addition, according to the present disclosure, the time for filtering out synthesizable molecules among the new substances generated by the molecular generative model and deriving the synthetic path may be shortened through a variational autoencoder-based machine learning methodology that may simultaneously generate the chemical structure and chemical reaction path (synthetic path) of a molecule having the required properties.

FIG. 2 is a flow chart illustrating an operation method of an autoencoding device for a synthetic molecule generative model according to an exemplary embodiment of the present disclosure.

An autoencoder is a device that learns a neural network model that learns a method of compressing a given data distribution into a latent space, or applies and utilizes a learned model. The autoencoder may include at least one encoder that compresses data and at least one decoder that decompresses data. In an exemplary embodiment, when input data is input to the encoder, the encoder may convert an image into a latent vector in the latent space, and the decoder may restore the latent vector back to an image to generate output data. In this case, the vector in the latent space that has undergone compression by the encoder is reduced in size compared to the input data, and the performance of the model may be known depending on how much the reduced input data is restored to the same size. The autoencoder may be implemented by the computing device described above.

In step S110, the autoencoding device may obtain a latent space including chemical reaction learning information. According to an exemplary embodiment of the present disclosure, the chemical reaction learning information may be information used for artificial intelligence learning as data on chemical reactions. The chemical reaction learning information may be commercially available chemical reaction data, reaction data including generally known reaction templates. The autoencoding device may convert the chemical reaction learning information into a latent space.

In step S130, the autoencoding device may obtain an embedding space that includes structural information of the molecule but omits the synthesis path information. In the case of the molecule generative model according to the present disclosure, structural information of molecules that are designed through an existing generative model but have optimized properties but do not know the synthesis path may be utilized as a condition.

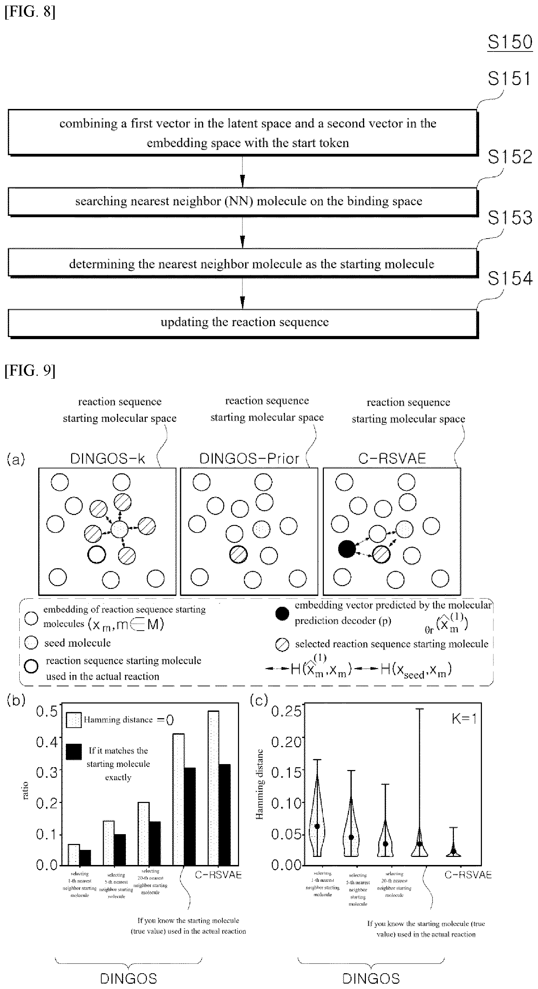

In step S150, the autoencoding device may sample and generate similar molecules from the first embedding space and the second embedding space through the generative model. Similar molecule sampling may mean response sampling based on seed molecule conditions. Step S150 will be described in more detail with reference to FIG. 8.

In step S170, the autoencoding device may generate an intermolecular synthesis path having seed structural information. In the present disclosure, the synthesis path may be interpreted in a similar meaning to an intermolecular reaction path. Step S170 may further include subroutines such as a step of sampling similar molecules from a latent space and an embedding space through a generative model, generating candidate molecules, and a step of generating an intermolecular synthesis path having seed structural information.

In step S190, the autoencoding device may output a molecular model including seed structural information and a synthetic pathway. According to the present disclosure, by simultaneously generating a chemical structure of a molecule having required properties and a chemical reaction pathway (synthetic pathway), the time for filtering out synthesizable molecules among the new substances generated by the generative model and deriving a synthetic pathway may be shortened.

FIG. 3 is a schematic diagram illustrating a process for generating chemical reaction data for a synthetic molecule generative model according to an exemplary embodiment of the present disclosure.

Referring to FIG. 3A, according to an exemplary embodiment of the present disclosure, chemical reaction data utilized for learning a molecular generative model may be constructed using about 150,000 molecules that are accessible or available for learning and 58 reaction templates that are mainly used for drug synthesis. In this case, among the total available molecules, about 130,000 molecules having a molecular weight of 100 to 300 g (grams)/mol (mol) may be utilized, and among these, 5,000 molecules may be randomly selected and utilized in the initial stage of the reaction.

Referring to FIG. 3B, molecules and reaction templates may be arbitrarily selected to generate reaction data. In an exemplary embodiment, a total of 2.1 million different chemical reaction data may be constructed, and each chemical reaction may be composed of up to three single reactions.

According to an exemplary embodiment of the present disclosure, the chemical reaction data includes a primary reactant and a partner reactant defined as a sequence, and the primary reactant and the partner reactant may be converted into a binary embedding vector.

According to an exemplary embodiment of the present disclosure, the chemical reaction data includes a reaction template defined as a sequence, and the reaction template may be converted into a one-hot vector.

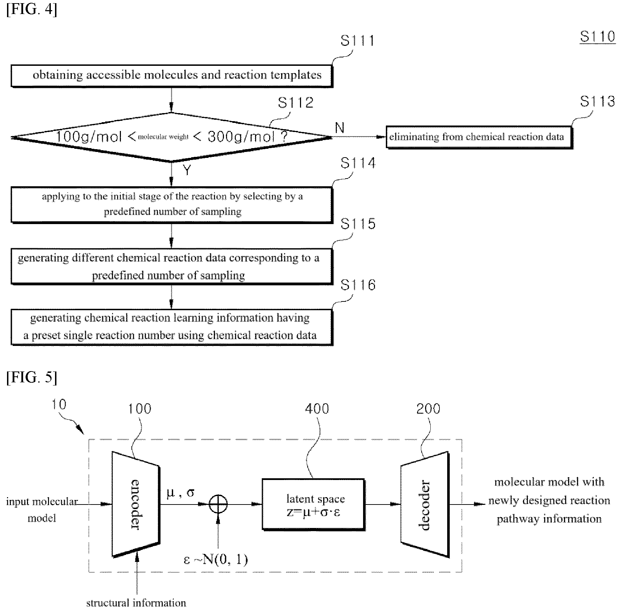

FIG. 4 is a flow chart illustrating a method for generating chemical reaction data for a synthetic molecule generative model according to an exemplary embodiment of the present disclosure.

As a step S110 of acquiring potential space, the following steps S111 to S116 may be performed.

In step S111, the autoencoding device may obtain accessible or learnable molecules and reaction templates. For example, according to an exemplary embodiment of the present disclosure, chemical reaction data utilized for learning a molecular generative model may be constructed using about 150,000 molecules that are previously accessible or available for learning and 58 reaction templates that are mainly utilized for drug synthesis. In this case, among the total available molecules, about 130,000 molecules having a molecular weight of 100 to 300 g (grams)/mol (mole) may be utilized.

In step S112, the autoencoding device may determine whether the molecular weight is within a predefined range. In the method for generating a molecular model using a computing device according to an exemplary embodiment of the present disclosure, the predefined range may be greater than 100 g/mol and less than 300 g/mol.

In step S113, the autoencoding device may remove molecules having molecular weights outside a predefined range from the chemical reaction data. For example, the autoencoding device may not store molecules having molecular weights smaller than 100 g/mol or larger than 300 g/mol in the chemical reaction data, or may remove the corresponding molecule data from the already stored chemical reaction data.

In step S114, the autoencoding device may be applied to the initial stage of the reaction by selecting a predefined number of samples. For example, the autoencoding device may be utilized in the initial stage of the reaction by selecting a number of sample molecules of 5,000. In step S115, the autoencoding device may generate different chemical reaction data corresponding to the predefined number of samples.

In step S116, the autoencoding device may generate chemical reaction learning information having a preset single reaction number using chemical reaction data. In an exemplary embodiment of the present disclosure, the preset single reaction number may be 3.

FIG. 5 is a schematic diagram illustrating an autoencoding device 10 for generating a syntheticable molecular model according to an exemplary embodiment of the present disclosure.

An autoencoding device 10 may include at least one encoder 100 for compressing data and at least one decoder 200 for decompressing data. In an exemplary embodiment, input data (MM) may be input to the encoder, and structural information (SI) of a molecule may be further input as a condition. In an exemplary embodiment, the encoder 100 may convert an image into a latent vector (z) on a latent space 400, and the decoder 200 may restore the latent vector (z) to generate output data (dMM). Here, the output data may include a reaction path.

The autoencoding device 10 according to the present disclosure may be an autoencoder that arranges and places data on a latent space 400 according to a predefined distribution. The autoencoding device 10 according to an exemplary embodiment may arrange and place data according to a normalized Gaussian distribution. According to an exemplary embodiment, the autoencoding device 10 may be an autoencoder that may arrange and place learning data having similar characteristics on a latent space so that the learning data are not arranged in a scattered manner on the latent space.

According to an exemplary embodiment of the present disclosure, the encoder 100 may transform input data (MM) into a latent vector (z) on a latent space. The input data (MM) may be classified to follow a normal distribution having a mean (μ) and a standard deviation (σ).

After encoding, the latent vector (z) may be added with noise (ε) that has the characteristic of following a standard normal distribution (i.e., a normal distribution with a mean of 0 and a standard deviation of 1).

In this case, the latent vector (z) may follow the following mathematical formula.

[Formula 1]

$$z = \mu + \sigma \cdot \varepsilon$$

Referring to mathematical expression 1, noise (ε) may function as a weight of variance.

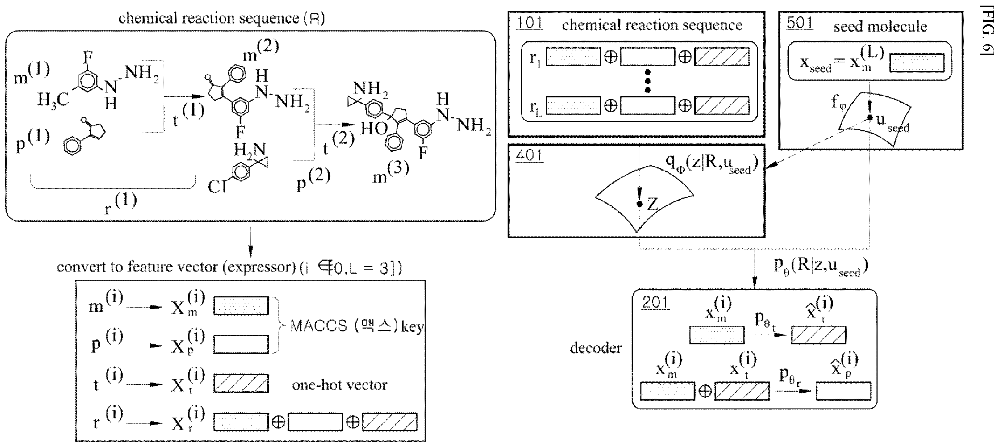

FIG. 6 is a conceptual diagram illustrating in detail the operation of an autoencoding device for a synthetic molecule generative model according to an exemplary embodiment of the present disclosure. Referring to FIG. 6, a generative model that may generate a reaction path by utilizing information on a target molecular structure as a condition according to the present disclosure is described. The molecular structure condition-based synthetic molecule generative model expressed in the present disclosure may be used interchangeably with a C-RSVAE (Conditional-Reaction Sequence Variational Autoencoder).

An autoencoding device for a synthesizable molecule generative model according to an exemplary embodiment of the present disclosure may include a memory for storing chemical reaction data, chemical reaction learning information, and structural information of a molecule, and a processor configured to learn an artificial neural network for implementing a molecule generative model based on the chemical reaction data, chemical reaction learning information, and structural information.

In the present disclosure, chemical reaction data may be defined in the form of a sequence. Therefore, a chemical reaction consisting of n single reactions is defined as a reaction sequence (R) as shown in the following mathematical expression 2.

[Formula 2]

$$R = \left[ r^{(0)}, r^{(1)}, \ldots, r^{(n)}, r^{(n+1=L)} \right]$$

Here, $r^{(i)}$ represents the i-th single reaction, i=0 represents the starting state of the reaction, and i=n+1=L represents the final state of the reaction.

Additionally, in the present disclosure, the primary reactant may be defined as $m^{(i)}$, the partner reactant may be defined as $P^{(i)}$, and the reaction template may be defined as $t^{(i)}$, respectively.

Both the primary reactant ($m^{(i)}$) and the partner reactant ($P^{(i)}$) may be represented by a binary embedding vector called MACCS key.

When expressed as an embedding vector, the primary reactant ($m^{(i)}$) vector may be expressed as $x_m^{(i)}$, the partner reactant ($P^{(i)}$) vector may be expressed as $x_p^{(i)}$, and the reaction template ($t^{(i)}$) may be expressed as a one-hot vector ($x_t^{(i)}$).

Through this, $r^{(i)}$ may be expressed as $x_r^{(i)}$ and may be obtained by concatenating $x_m^{(i)}$, $x_p^{(i)}$, $x_t^{(i)}$ (i.e., $x_r^{(i)} = X_m^{(i)} \oplus X_p^{(i)} \oplus X_t^{(i)}$)

In an exemplary embodiment, the above expression may be defined as $X_m^{(0)} = 0,\ X_p^{(0)} = X_m^{(1)}$ for i=0, where $X_t^{(0)}$ may be defined as a start token.

In an exemplary embodiment, the above expression may be defined as $X_p^{(L)} = 0$ if i=L, and $X_t^{(L)}$ may be defined as a end token.

In this disclosure, a chemical reaction may be used as an input value of a generative model. In the learning process, the final product of a given chemical reaction may be defined as a seed molecule (seed). In contrast, an arbitrary molecule may be utilized in the testing process.

The molecular generative model may include a reaction sequence encoder ($q_\Phi(z|R, u_{seed})$) 101 that generates a latent space 401 based on chemical reaction learning information, a seed molecule encoder ($f_\varphi(X_{seed})$) that generates an embedding space 501 based on seed structural information for a seed molecule, which is a final product of a chemical reaction, and a decoder ($p_\theta(R|z, u_{seed})$) 201 that outputs a reconstructed reaction sequence by decoding a reaction molecule prediction neural network and a reaction template prediction model generated from the latent space 401 and the embedding space 501.

Here, φ, θ, Φ are all model parameters that are optimized through learning. And F(•) is defined as an arbitrary multilayer neural network.

$f_\varphi$ is composed of a neural network and calculates the seed molecule embedding ($u_{seed}$) (i.e., $u_{seed} = f_\varphi(x_{seed})$), and the calculated $u_{seed}$ is used as a condition when calculating the latent vector by encoding the chemical reaction.

The reaction sequence encoder ($q_\Phi(z|R, u_{seed})$) 101 encodes the calculated seed molecule embedding($u_{seed}$) and the chemical reaction (R) to calculate the latent vector (Z), and recognizes the chemical reaction steps as data in the form of a sequence to calculate the embedding vector for the entire reaction through a bidirectional gated recurrent unit, which is a type of RNN (Recurrent Neural Network).

In this case, according to the definition of variational autoencoder (VAE), the latent vector (Z) is modeled as a normal distribution, and the mean (μ) and standard deviation (σ) are calculated as in the following mathematical expression 3.

[Formula 3]

$$[\mu, \sigma] = F_h\left(\text{BiGRU}\left(\left[x_r^{(1)}, x_r^{(2)}, \dots, x_r^{(L)}\right]\Big|u_{seed}\right)\right)$$

Bidirectional GRU (BiGRU) is a model that recognizes a given sequence in both directions and calculates an embedding vector, and $F_h$ is a neural network that calculates the mean (μ) and standard deviation (σ) using the previously calculated embedding vector.

According to an exemplary embodiment of the present disclosure, the reaction sequence encoder 101 may use the embedding vector as a condition when calculating the latent vector on the latent space 401.

According to an exemplary embodiment of the present disclosure, the reaction sequence encoder 101 may generate a latent vector as a result of encoding the chemical reaction of the embedding vector and the seed molecule.

According to an exemplary embodiment of the present disclosure, a reaction sequence encoder 101 may generate a latent embedding vector for an entire chemical reaction by learning chemical reaction data defined as a sequence based on a recurrent neural network.

According to an exemplary embodiment of the present disclosure, the reactive sequence encoder 101 may generate a latent embedding vector using a bidirectional gated recurrent unit, which is a recurrent neural network that recognizes a given sequence in both directions and is designed to include a forgetting gate.

According to an exemplary embodiment of the present disclosure, a seed molecule encoder ($f_\varphi(x_{seed})$) may provide an embedding vector encoding a chemical reaction of a seed molecule to a reaction sequence encoder 101.

The decoder ($p_\theta(R|z, u_{seed})$) 201 may be composed of two types of neural networks: a response template prediction neural network ($P\theta_t$) and a reaction-composing molecule prediction neural network ($P\theta_r$). Here, $\theta_t$, $\theta_r$ are each model parameters obtained through learning.

A reaction template prediction neural network ($P\theta_t$) may predict a template ( $x_t^{(i)}$ ) for a given primary reactant ( $x_m^{(i)}$ ).

A molecular prediction neural network ($P\theta_r$) predicts the primary reactant ( $x_m^{(1)} = x_p^{(0)}$ ) used in the initial step of a reaction and the partner reactant ($x_p^{(i>0)}$) for a given primary reactant ($x_m^{(i>0)}$) and reaction template ($x_t^{(i>0)}$).

That is, the predicted template ($\hat{X}_t^{(j)}$) and reactant ($\hat{X}_p^{(k)}$) may be calculated as in the following mathematical equations 4 and 5.

[Formula 4]

$$\hat{x}_p^{(j)} \sim p_{\theta_t}\left(m^{(j)}|z, u_{seed}\right) = \text{Softmax}\left[F_t\left(\text{GRU}\left(x_m^{(j)}|z, u_{seed}\right)\right)\right]$$

[Formula 5]

$$\hat{x}_p^{(j)} \sim p_{\theta_r}\left(m^{(k)}, t^{(k)}|z, u_{seed}\right) = \text{Sigmoid}\left[F_p\left(\text{GRU}\left(x_m^{(k)}\oplus x_t^{(k)}|z, u_{seed}\right)\right)\right]$$

Here, j and k are integers satisfying $j \in [1, L]$ and $k \in [0, L-1]$, respectively, and $F_t$ and $F_p$ are neural network models that receive the output values of the GRU obtained in the 1st or k-th step as input values, respectively.

In the present disclosure, when generating a new molecule using a learned artificial neural network, the encoder may be omitted and only the learned decoder ($p_\theta (R|z, u_{seed})$) 201 may be utilized.

According to an exemplary embodiment of the present disclosure, the decoder 201 may include a reaction template prediction neural network, and a reaction-constituting molecule prediction neural network.

According to an exemplary embodiment of the present disclosure, a reaction template prediction neural network may be characterized by being designed to predict a reaction template for a primary reactant that is a prediction target.

According to an exemplary embodiment of the present disclosure, a molecular prediction neural network may be characterized by being designed to predict a primary reactant as a prediction target, a partner reactant for a reaction template, and a starting primary reactant utilized in a reaction initiation step.

According to an exemplary embodiment of the present disclosure, a latent vector may be modeled according to a normal distribution having a mean and a standard deviation, and the mean and the standard deviation may be characterized as being calculated through a latent embedding vector.

According to an exemplary embodiment of the present disclosure, each of a synthetic pathway and a molecular structure may be mapped onto a latent space, and a molecule may be generated by reconstructing the synthetic pathway from latent vectors on the two latent spaces.

According to an exemplary embodiment of the present disclosure, since the decoder 201 sequentially creates a synthesis path to generate a molecule, all molecules generated may have a synthesis path.

Therefore, the autoencoding device according to the exemplary embodiment of the present disclosure may generate a molecule similar to the seed molecule and having a synthetic path by utilizing the generative model when the synthesis of the seed molecule is difficult.

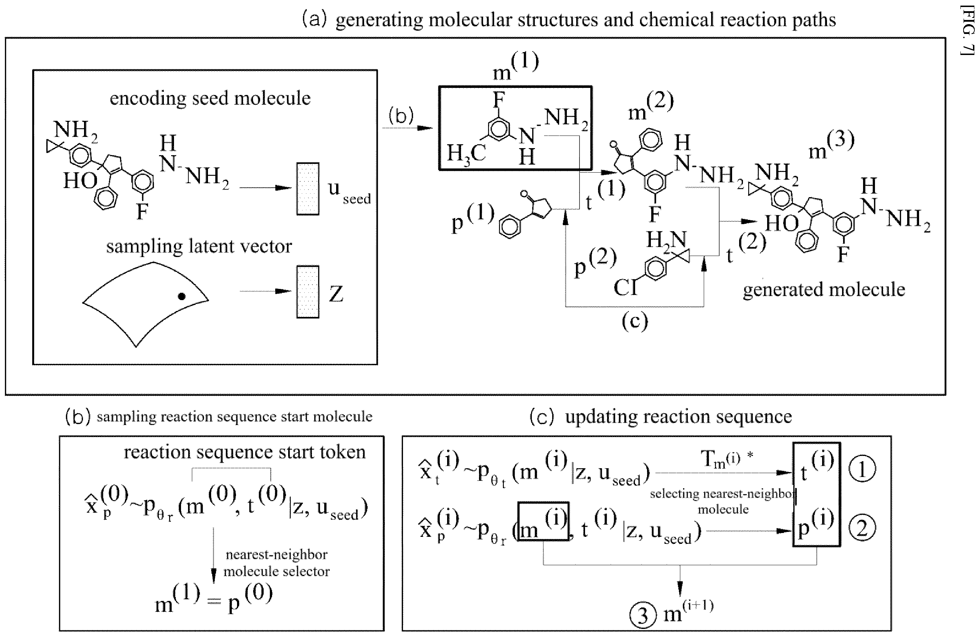

FIG. 7 is a conceptual diagram illustrating in detail the operation of an autoencoding device for a synthetic molecule generative model according to an exemplary embodiment of the present disclosure.

Referring to FIG. 7, sampling of new synthesizable molecules may be performed by utilizing the embedding vector ($u_{seed}$) for the seed molecule (seed) and the latent vector (Z) sampled from the latent space modeled as a normal distribution, and may be separated into the process of sampling the starting molecule (FIG. 7B) and updating the reaction sequence (FIG. 7C).

According to the present disclosure, since the reaction is formed with a molecule that is already learned or available, sampling may be performed based on the distance between the predicted result and the available molecule through the decoder. That is, the starting molecule ($m^{(1)}$) is defined as the following mathematical expression 6.

[Formula 6]

$$\left[m_1^{(1)}, \ldots, m_k^{(1)}\right] \sim \text{kNN}_{m \in M}\left[H\left(\hat{x}_m^{(1)}, x_m\right) + H(x_{seed}, x_m)\right]$$

In this case, $\hat{x}_m^{(1)} \sim p_{\theta_r}\left(m^{(0)}, t^{(0)}|z, u_{seed}\right)$ is the predicted result through the decoder, and $\text{kNN}_m$ may

represent the k-nearest-neighbor sampling for the set (M) consisting of the predicted result $\left(\hat{x}_m^{(1)}\right)$ and the starting molecules defined in advance. The Hamming distance (H) may be used as a criterion for defining the adjacent neighbors.

In this case, rather than simply utilizing the first Hamming distance ( $H\left(\hat{x}_m^{(1)}, x_m\right)$ ) with the predefined molecule, the second Hamming distance ($H(x_{seed}, x_m)$) with the actual seed molecule may be additionally considered.

Referring to FIG. 7C, the process of updating the reaction sequence consists of a process of predicting a reaction template ($t^{(i)}$) and a step of predicting a partner reactant ($P^{(i)}$), and may be expressed as follows.

[Formula 7]

$$t^{(i)} \sim T_m(i) * \hat{x}_t^{(i)}$$

[Formula 8]

$$\left[p_1^{(i)}, \ldots, p_k^{(i)}\right] \sim kNN_{p \in P[t^{(i)}]}\left[H\left(\hat{x}_p^{(i)}, x_p\right)\right]$$

wherein, $\hat{x}_t^{(i)} \sim p_{\theta_t}\left(m^{(i)} \big| z, u_{seed}\right), \hat{x}_p^{(i)} \sim p_{\theta_r}\left(m^{(i)}, t^{(i)} \big| z, u_{seed}\right)$ .

In an exemplary embodiment, when predicting a reaction template ($t^{(i)}$), sampling is performed only for reactions applicable to a given primary reactant ($m^{(i)}$), and for this purpose, a mask vector defined as $T_m^{(i)}$ may be used.

In an exemplary embodiment, when predicting a partner reactant ($P^{(i)}$), it may be inferred through k-nearest-neighbor sampling ($kNN_{P[t^{(i)}]}$) between the predicted result $\left(\hat{x}_p^{(i)}\right)$ and the set ($P[t^{(i)}]$) of molecules applicable to a specific reaction template ($t^{(i)}$), similar to the case of sampling the previous starting molecule, via the decoder. This reaction sequence update may be repeated until the predicted reaction template is a 'end token' or the maximum number of reaction steps (= 3) is reached.

According to the present disclosure, an autoencoding device may generate a chemical reaction through sampling of seed molecules and reaction latent spaces by utilizing a learned generative model.

FIG. 8 is a conceptual diagram illustrating in detail the operation of an autoencoding device for a synthetic molecule generative model according to an exemplary embodiment of the present disclosure. Any description of the operation of FIG. 8 that overlaps with that of FIG. 7 will be omitted.

FIG. 8 is a conceptual diagram illustrating in detail the operation of an autoencoding device for a synthetic molecule generative model according to an exemplary embodiment of the present disclosure. Any description of the operation of FIG. 8 that overlaps with that of FIG. 7 will be omitted.

In step S151, the autoencoding device may combine a first vector in the latent space and a second vector in the embedding space with the start token.

In step S152, the nearest neighbor (NN) molecule may be searched on the binding space. According to an exemplary embodiment of the present disclosure, the autoencoding device may calculate a first Hamming distance between the predicted molecule and the predefined starting molecule through the decoder based on the Hamming distance. According to an exemplary embodiment of the present disclosure, a second Hamming distance between the starting molecule and the seed molecule may be calculated.

In an exemplary embodiment, the Hamming distance (H) is an indicator of the similarity of molecules, and the smaller the distance, the more similar the properties may be.

In step S153, the autoencoding device may determine the nearest neighbor molecule as the starting molecule of the chemical reaction. In step S154, the autoencoding device may update the reaction sequence.

FIG. 9 is a conceptual diagram illustrating the operation of an autoencoding device for a synthetic molecule generative model according to an exemplary embodiment of the present disclosure.

Referring to FIG. 9, the performance resulting from the application of the learned molecular generative model is explained.

FIG. 9A is a conceptual diagram of the model used to compare restoration performance.

In order to verify the in-domain seed molecule restoration performance of the model proposed in this disclosure, a comparison was performed with DINGOS, a methodology for generating chemical reactions capable of generating molecules similar to seed molecules through conventional machine learning methods and heuristic rule-based methods.

In an exemplary embodiment, the in-domain seed molecules were randomly extracted from 1,000 data that were not used for learning among chemical reaction data (reaction database), and the restoration performance was compared.

A comparison group based on a total of four existing models was set up (DINGOS-1, DINGOS-5, DINGOS-20, DINGOS-Prior). The DINGOS-k (k=1,5,20) method means selecting k molecules most similar to the seed molecule in the process of selecting the starting molecule in the DINGOS model, and in the case of DINGOS-Prior, since the chemical reaction information for the seed molecule is already known, the starting molecule corresponding to the actual true value is selected to form the chemical reaction.

The evaluation of the restoration performance was performed by utilizing the Hamming distance between the most similar molecule among the molecules obtained from each method and the seed molecule, and the ratio of cases where the value is 0 (cases where the same substructure information is present) and cases where the molecule is restored to be completely identical was calculated. In this case, when both the encoder and decoder proposed in this disclosure were utilized, a restoration rate of 98% was achieved for 1,000 data.

FIG. 9B shows the ratio of molecules generated by the DINGOS method in which the Hamming distance to the seed molecule is 0 or is completely identical to the existing seed molecule.

As may be seen in FIG. 9B, in the case of the DINGOS-k based method, it was confirmed that as k increased, more similar molecules were generated, but in the case of DINGOS-Prior, it was confirmed that the ratio increased by 2 to 7 times. This may be interpreted to mean that utilizing only the similarity with the existing seed molecule is insufficient for actually generating molecules similar to the seed molecule.

FIG. 9C shows the distribution of Hamming distances for the seed molecules of the generated molecules. Even when examining the distribution of Hamming distances between the actually generated molecules and the seed molecules, it is confirmed that molecules more similar to the seed molecule are obtained in DINGOS-Prior.

From this perspective, it may be confirmed that the molecule generation method (C-RSVAE) proposed in this disclosure outperforms the DINGOS-k based method and shows similar performance to DINGOS-Prior even though it does not provide information on the chemical reaction for the actual test molecule. In other words, it has been proven that by utilizing the latent space learned through this disclosure, it is possible to generate molecules similar to the seed molecules of the existing in-domain region more effectively.

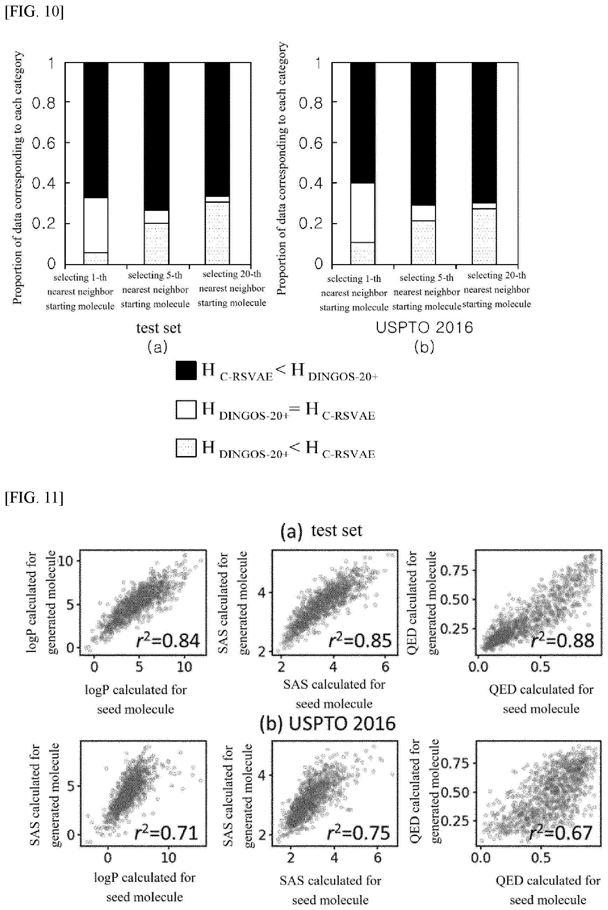

FIG. 10 is a graph showing the performance of an autoencoding device for a synthesizable molecule generative model according to an exemplary embodiment of the present disclosure.

Referring to FIG. 10, the Hamming distance criterion performance between the generated molecule and the seed molecule is compared between the compound reactive volatilization (CRSVAE) methodology and the existing methods (e.g., DINGOS).

The main application of the methodology (C-RSVAE) proposed in this disclosure is the generation of new synthesizable molecules having structural information similar to that of the seed molecule, so it is necessary to evaluate the performance when seed molecules existing in the out-of-domain as well as in the actual in-domain are given as conditions. In the case of the in-domain, 1,000 new seed molecules that were not used for learning were sampled similarly to FIG. 9, and in the case of the out-of-domain, 1,000 seed molecules were randomly sampled and used among the molecules registered in 2016 at the USPTO, which has reaction information on molecules registered as patents until 2016 (i.e., "USPTO 2016"). In this case, DINGOS-20+ was used as the reference model, which is a method of additionally sampling starting molecules when the number of sampled reactions is less than 300 in the case of DINGOS-20.

Among the molecules generated for performance comparison, the Hamming distance to the seed molecule was calculated, and the average value of the closest K (=1, 5, 20) molecules was calculated for each method of DINGOS-20+ and C-RSVAE (i.e., HDINGOS-20+, HC-RSVAE). Then, the magnitude of each calculated value was classified into three categories and compared and displayed. (i.e., $H_{C\text{-RSVAE}} < H_{DINGOS\text{-}20}+$ (black), $H_{DINGOS\text{-}20+} = H_{C\text{-RSVAE}}$ (white), $H_{DINGOS\text{-}20+} < H_{C\text{-RSVAE}}$ (dot pattern)).

As may be seen in FIG. 10, when utilizing the methodology (C-RSVAE) proposed in this disclosure, the rate of generating molecules more similar to the seed molecule is high, and this proves that it is efficient to learn structural information and reaction information together.

FIG. 11 is a graph showing the performance of an autoencoding device for a synthesizable molecule generative model according to an exemplary embodiment of the present disclosure.

Referring to FIG. 11, the correlation between the molecular properties (logP, SAS, QED) generated by the model proposed in the present disclosure and the seed molecule is shown.

Based on the previous results, three properties of logP, SAS, and QED were calculated for the molecules generated through the model proposed in this disclosure, and the correlation with the seed molecule was compared. As may be seen in FIG. 11, both the test data and USPTO 2016 showed a high correlation. This relationship is consistent with the structure-property relationship that has been mainly accepted in the past.

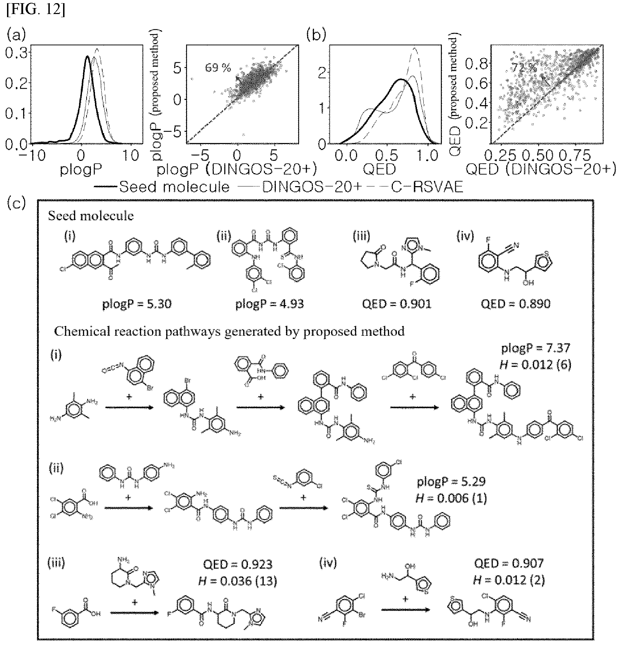

FIG. 12 is a conceptual diagram illustrating the performance of an autoencoding device for a synthesizable molecule generative model according to an exemplary embodiment of the present disclosure.

FIG. 12A and FIG. 12B are graphs comparing the distribution of properties (plogP, QED) of generated molecules and seed molecules, and FIG. 12C is a conceptual diagram showing the results of molecule generation that may reveal a new synthetic route based on a virtual molecule with optimized properties obtained through an existing generative model.

As may be seen in FIG. 12A and FIG. 12B, most of the generated molecules may have improved property values (plogP, QED) than the seed molecule. This may be interpreted as the fact that the proposed method may obtain molecules with improved property values because random reactions are obtained from a given seed molecule and a randomly sampled latent vector. **In** addition, through comparison with DINGOS-20+ and the method proposed in this disclosure, it may be confirmed that molecules with improved properties are obtained at a high rate in this disclosure, which may be interpreted as the increase in sampling diversity due to random sampling in the latent space where chemical reactions are learned.

**In** an exemplary embodiment, if the proposed model is used to generate a new synthesizable molecule based on a virtual molecule with existing optimized properties, it may be confirmed that a new molecule having a synthetic route with improved property values than the existing optimized molecule may be obtained for each of plogP and QED.

The method according to the present disclosure described above may be implemented as a program (or application) to be executed by being combined with a hardware server and stored on a medium. The disclosed embodiments may be implemented in the form of a recording medium storing instructions executable by a computer. The instructions may be stored in the form of program codes, and when executed by a processor, may generate a program module to perform the operations of the disclosed embodiments. The recording medium may be implemented as a computer-readable recording medium.

A computer-readable recording medium includes all types of recording media storing instructions that may be deciphered by a computer. **In** an exemplary embodiment, the recording medium may store a computer program in the form of a computer program code or an executable file. The program may include codes coded in a computer language, such as C, C++, JAVA, or machine language, that may be read by the processor (CPU) of the computer through the device interface of the computer so that the computer reads the program and executes the methods implemented as the program. Such codes may include functional codes related to functions that define functions necessary for executing the methods, and may include control codes related to execution procedures necessary for the processor of the computer to execute the functions according to a predetermined procedure. **In** addition, such codes may further include memory reference-related codes regarding which location (address address) of the internal or external memory of the computer should be referenced for additional information or media necessary for the processor of the computer to execute the functions. In addition, if the processor of the computer needs to communicate with another computer or server located remotely in order to execute the above functions, the code may further include communication-related code regarding how to communicate with another computer or server located remotely using the communication module of the computer, and what information or media to send and receive during communication.

The above-mentioned storage medium means a medium that stores data semi-permanently and may be read by a device, rather than a medium that stores data for a short period of time, such as a register, cache, or memory. Specifically, examples of the above-mentioned storage medium include, but are not limited to, ROM, RAM, CD-ROM, magnetic tape, floppy disk, or optical data storage device. That is, the above-mentioned program may be stored in various storage media on various servers that the computer may access or in various storage media on the user's computer. In addition, the above-mentioned medium may be distributed to computer systems connected to a network, so that a computer-readable code may be stored in a distributed manner.

The steps of a method or algorithm described in connection with the embodiments of the present disclosure may be implemented directly in hardware, implemented in a software module executed by hardware, or implemented by a combination of these. The software module may reside in a Random Access Memory (RAM), a Read Only Memory (ROM), an Erasable Programmable ROM (EPROM), an Electrically Erasable Programmable ROM (EEPROM), a flash memory, a hard disk, a removable disk, a CD-ROM, or any other form of computer-readable recording medium well known in the art to which the present disclosure pertains.

As described above, the disclosed embodiments have been described with reference to the attached drawings. Those skilled in the art to which the present disclosure pertains will understand that the present disclosure may be implemented in forms other than the disclosed embodiments without changing the technical idea or essential features of the present disclosure. The disclosed embodiments are exemplary and should not be construed as limiting.

**Claims**

1. An autoencoding device for a synthesizable molecular generative model, comprising:

  a memory storing chemical reaction data, chemical reaction learning information, and molecular structure information; and
  a processor configured to learn a neural network that implements a molecular generative model based on the chemical reaction data, the chemical reaction learning information, and the structural information, wherein the molecular generative model comprises:

a reaction sequence encoder that generates a latent space based on the chemical reaction learning information;
a seed molecule encoder that generates an embedding space based on seed structural information for the seed molecule that is a final product of the chemical reaction; and
a decoder that outputs a reconstruction reaction sequence by decoding a reaction molecule predictive neural network and a reaction template prediction model generated from the latent space and the embedding space.

2. The autoencoding device for a synthesizable molecular generative model of claim 1, wherein:

the seed molecule encoder is **characterized by**
providing an embedding vector encoding the chemical reaction of the seed molecule to the reaction sequence encoder.

3. The autoencoding device for a synthesizable molecular generative model of claim 2, wherein:

the reaction sequence encoder is **characterized by**
using the embedding vector under a condition when calculating the latent vector in the latent space.

4. The autoencoding device for a synthesizable molecular generative model of claim 2, wherein:

the reaction sequence encoder is **characterized by**
generating a latent vector as a result of encoding the embedding vector and the chemical reaction of the seed molecule.

5. The autoencoding device for a synthesizable molecular generative model of claim 4, wherein:

the reaction sequence encoder is **characterized by**
generating a latent embedding vector for the entire chemical reaction by learning the chemical reaction data defined as a sequence based on a recurrent neural network.

6. The autoencoding device for a synthesizable molecular generative model of claim 5, wherein:

the reaction sequence encoder is **characterized by**
generating the latent embedding vector using a bidirectional gated recurrent unit, a model designed to recognize a given sequence bidirectionally and comprise a forget gate, as the recurrent neural network.

7. The autoencoding device for a synthesizable molecular generative model of claim 5, wherein:

the latent vector is **characterized by**
being modeled according to a normal distribution with a mean and standard deviation.

8. The autoencoding device for a synthesizable molecular generative model of claim 7, wherein:

the mean and the standard deviation is **characterized by**
being calculated through the latent embedding vector.

9. The autoencoding device for a synthesizable molecular generative model of claim 1, wherein:

the decoder is **characterized by**
comprising a reaction template predictive neural network, and a molecular predictive neural network that constitutes the reaction.

10. The autoencoding device for a synthesizable molecular generative model of claim 9,
wherein:

the reaction template predictive neural network is **characterized by**
being designed to predict a reaction template for the primary reactant to be predicted.

11. The autoencoding device for a synthesizable molecular generative model of claim 9,
wherein:

the molecular predictive neural network is **characterized by**
being designed to predict a partner reactant for the primary reactant to be predicted and reaction template, and the initial primary reactant utilized in the reaction initiation step.

12. The autoencoding device for a synthesizable molecular generative model of claim 1, wherein:
it is characterized that:

the chemical reaction data comprises a primary reactant and a partner reactant defined as a sequence, and the primary reactant and the partner reactant are converted into binary embedding vectors.

13. The autoencoding device for a synthesizable molecular generative model of claim 1,
wherein:

it is characterized that
the chemical reaction data comprises a reaction template defined as a sequence, and the reaction template is converted into a one-hot vector.

14. A method of generating a molecular model using a computing device, comprising:

obtaining a latent space comprising chemical reaction learning information;
obtaining an embedding space comprising structural information but omitting synthetic path information of a molecule;
sampling similar molecules from the latent space and the embedding space through a generative model, and generating candidate molecules;
generating a synthetic pathway between molecules with seed structural information; and
outputting a seed molecular model that comprises the seed structural information and the synthetic pathway.

15. The method of generating a molecular model using a computing device of claim 14, wherein:
it is characterized that
the obtaining the latent space comprises:

obtaining a learnable molecule and a reaction template;
determining whether the molecular weight is within a predefined range; and
removing molecules having a molecular weight outside the predefined range from the chemical reaction data.

16. The method of generating a molecular model using a computing device of claim 15, wherein:

it is characterized that
the obtaining the latent space further comprises:

selecting a predefined number of samples to apply at the initial reaction stage;
generating different chemical reaction data corresponding to the predefined number of samples; and
generating chemical reaction learning information with a preset number of single reactions using the chemical reaction data.

17. The method of generating a molecular model using a computing device of claim 16, wherein:
the preset number of single reactions is **characterized by** being three.

18. The method of generating a molecular model using a computing device of claim 15, wherein:
the predefined range is **characterized by** being greater than 100 g(gram)/mol(mole) and less than 300 g/mol.

19. The method of generating a molecular model using a computing device of claim 14, wherein:

it is characterized that
the sampling similar molecules and generating candidate molecules comprises:

combining a first vector **in** the latent space and a second vector **in** the embedding space with a start token;
searching for nearest-neighbor (NN) molecules **in** the combined space;
determining the nearest-neighbor molecule as the starting molecule of the chemical reaction; and
updating the reaction sequence.

20. The method of generating a molecular model using a computing device of claim 19, wherein:
the searching for nearest-neighbor (NN) molecules **in** the combined space comprises:

calculating a first Hamming distance between the predictive molecule predicted through the decoder and the predefined starting molecule based on the Hamming distance; and
calculating a second Hamming distance between the starting molecule and the seed molecule.

[FIG. 1]

[FIG. 2]

[FIG. 3]

[FIG. 4]

S110

S111

obtaining accessible molecules and reaction templates

S112

$100g/mol < $ molecular weight $ < 300g/mol$ ? → N → eliminating from chemical reaction data — S113

Y

S114

applying to the initial stage of the reaction by selecting by a predefined number of sampling

S115

generating different chemical reaction data corresponding to a predefined number of sampling

S116

generating chemical reaction learning information having a preset single reaction number using chemical reaction data

[FIG. 5]

10    100    400    200

input molecular model → encoder → $\mu$, $\sigma$ → ⊕ → latent space $z=\mu+\sigma\cdot\varepsilon$ → decoder → molecular model with newly designed reaction pathway information

$\varepsilon \sim N(0, 1)$

structural information

[FIG. 6]

(a) generating molecular structures and chemical reaction paths

encoding seed molecule

sampling latent vector

generated molecule

(b) sampling reaction sequence start molecule

reaction sequence start token

$$\hat{x}_p^{(0)} \sim p_{\theta_r}(m^{(0)}, t^{(0)} | z, u_{seed})$$

nearest-neighbor molecule selector

$$m^{(1)} = p^{(0)}$$

(c) updating reaction sequence

$$\hat{x}_t^{(i)} \sim p_{\theta_t}(m^{(i)} | z, u_{seed}) \xrightarrow{T_{m^{(i)}} *} t^{(i)} \quad ①$$

selecting nearest-neighbor molecule

$$\hat{x}_p^{(i)} \sim p_{\theta_r}(m^{(i)}, t^{(i)} | z, u_{seed}) \longrightarrow p^{(i)} \quad ②$$

$$③ \ m^{(i+1)}$$

[FIG. 7]

[FIG. 8]

S150

S151

combining a first vector in the latent space and a second vector in the embedding space with the start token

S152

searching nearest neighbor (NN) molecule on the binding space

S153

determining the nearest neighbor molecule as the starting molecule

S154

updating the reaction sequence

[FIG. 9]

reaction sequence starting molecular space

reaction sequence starting molecular space

reaction sequence starting molecular space

(a)  DINGOS-k    DINGOS-Prior    C-RSVAE

○ embedding of reaction sequence starting molecules $(x_m, m \in M)$

○ seed molecule

○ reaction sequence starting molecule used in the actual reaction

● embedding vector predicted by the molecular prediction decoder (p) $\text{or } (\hat{x}_m^{(1)})$

⊘ selected reaction sequence starting molecule

$-\cdot-H(\hat{x}_m^{(1)}, x_m) \cdot - H(x_{seed}, x_m)$

(b)

Hamming distance $= 0$

If it matches the starting molecule exactly

ratio

0.5
0.4
0.3
0.2
0.1
0.0

selecting 1-th nearest neighbor starting molecule | selecting 5-th nearest neighbor starting molecule | selecting 20-th nearest neighbor starting molecule | C-RSVAE

If you know the starting molecule (true value) used in the actual reaction

DINGOS

(c)

Hamming distance

0.25
0.20
0.15
0.10
0.05
0.00

K=1

selecting 1-th nearest neighbor starting molecule | selecting 5-th nearest neighbor starting molecule | selecting 20-th nearest neighbor starting molecule | C-RSVAE

If you know the starting molecule (true value) used in the actual reaction

DINGOS

[FIG. 10]

test set
(a)

USPTO 2016
(b)

$H_{C\text{-}RSVAE} < H_{DINGOS\text{-}20+}$

$H_{DINGOS\text{-}20+} = H_{C\text{-}RSVAE}$

$H_{DINGOS\text{-}20+} < H_{C\text{-}RSVAE}$

[FIG. 11]

(a) test set

(b) USPTO 2016

[FIG. 12]

## INTERNATIONAL SEARCH REPORT

| International application No. |
| --- |
| **PCT/KR2023/005431** |

| A. | CLASSIFICATION OF SUBJECT MATTER |
| --- | --- |
| | **G16C 20/70**(2019.01)i; **G16C 20/50**(2019.01)i; **G16C 20/10**(2019.01)i; **G16C 20/30**(2019.01)i; **G16C 20/90**(2019.01)i; **G06N 3/04**(2006.01)i; **G16C 20/62**(2019.01)i; **G16C 60/00**(2019.01)i |
| | According to International Patent Classification (IPC) or to both national classification and IPC |

| B. | FIELDS SEARCHED |
| --- | --- |
| | Minimum documentation searched (classification system followed by classification symbols) |
| | G16C 20/70(2019.01); G06N 20/00(2019.01); G06N 3/08(2006.01); G16C 20/10(2019.01); G16C 20/30(2019.01); G16C 20/50(2019.01) |
| | Documentation searched other than minimum documentation to the extent that such documents are included in the fields searched |
| | Korean utility models and applications for utility models: IPC as above<br>Japanese utility models and applications for utility models: IPC as above |
| | Electronic data base consulted during the international search (name of data base and, where practicable, search terms used) |
| | eKOMPASS (KIPO internal) & keywords: 화학반응(chemical reaction), 구조정보(structure information), 목표 분자(target molecule), 신경망(neural network), 인코더(encoder), 디코더(decoder), 오토인코딩 장치(autoencoder) |

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2021-084234 A1 (BENEVOLENTAI TECHNOLOGY LIMITED) 06 May 2021 (2021-05-06)<br>See paragraph [0039]; claims 1-3; and figure 1. | 1-20 |
| A | US 2021-0374551 A1 (INTERNATIONAL BUSINESS MACHINES CORPORATION) 02 December 2021 (2021-12-02)<br>See entire document. | 1-20 |
| A | KR 10-2022-0022059 A (ARONTIER CO., LTD.) 24 February 2022 (2022-02-24)<br>See entire document. | 1-20 |
| A | GRIFFITHS, R.-R. et al. Constrained Bayesian optimization for automatic chemical design using variational autoencoders. Chemical Science. 2020, vol. 11, pp. 577–586.<br>See entire document. | 1-20 |
| A | LIM, J. et al. Molecular generative model based on conditional variational autoencoder for de novo molecular design. Journal of Cheminformatics. 2018, vol. 10, document no. 31, pp. 1-9.<br>See entire document. | 1-20 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **20 July 2023** | **20 July 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/005431**

| Patent document cited in search report | | | Publication date (day/month/year) | Patent family member(s) | | | Publication date (day/month/year) |
|---|---|---|---|---|---|---|---|
| WO | 2021-084234 | A1 | 06 May 2021 | CN | 114600194 | A | 07 June 2022 |
| | | | | EP | 4052261 | A1 | 07 September 2022 |
| | | | | US | 2022-0406412 | A1 | 22 December 2022 |
| US | 2021-0374551 | A1 | 02 December 2021 | None | | | |
| KR | 10-2022-0022059 | A | 24 February 2022 | KR | 2022-0062485 | A | 17 May 2022 |

Form PCT/ISA/210 (patent family annex) (July 2022)